# EUROPEAN PATENT SPECIFICATION

(11) **EP 3 147 351 B1**
(45) Date of publication and mention of the grant of the patent: **28.08.2019**
(21) Application number: 15795904.0
(22) Date of filing: 14.04.2015
(51) Int. Cl.: C12P 13/08, C12P 13/22

(54) **MICROORGANISM HAVING IMPROVED INTRACELLULAR ENERGY LEVEL AND METHOD FOR PRODUCING L-AMINO ACID USING SAME**
MIKROORGANISMUS MIT VERBESSERTEM INTRAZELLULÄREN ENERGIENIVEAU UND VERFAHREN ZUR HERSTELLUNG VON L-AMINOSÄURE DAMIT
MICROORGANISME PRÉSENTANT UN NIVEAU D'ÉNERGIE INTRACELLULAIRE AMÉLIORÉ ET PROCÉDÉ DE PRODUCTION D'ACIDES AMINÉS L À L'AIDE DE CE MICROORGANISME

(30) Priority: 23.05.2014 KR 20140062593
(43) Date of publication of application: 29.03.2017
(73) Proprietor: CJ Cheiljedang Corporation, Seoul 100-400 (KR)
(72) Inventor: JANG, Juno, Seoul 153-810 (KR); PARK, Hye Min, Seoul 157-801 (KR); LEE, Kwang Ho, Seoul 157-840 (KR); LEE, Keun Cheol, Hwaseong-si Gyeonggi-do 445-320 (KR); HONG, Hyeong Pyo, Gangneung-si Gangwon-do 210-948 (KR)
(74) Representative: Potter Clarkson
(86) International application number: PCT/KR2015/003625
(87) International publication number: WO 2015/178586

(56) References cited:
- EP-A1- 1 829 965
- WO-A2-02/077183
- WO-A2-2006/034005
- WO-A2-2007/024756
- KR-A- 20070 086 634
- KR-A- 20130 080 930
- KR-B1- 100 792 095
- MADEMIDIS A ET AL: "Transport activity of FhuA, FhuC, FhuD and FhuB derivatives in a system free of polar effects, and stoichiometry of components involved in ferrichrome uptake", MOLECULAR AND GENERAL GENETICS, vol. 258, no. 1-2, April 1998 (1998-04), pages 156-165, XP002767999, ISSN: 0026-8925
- DATABASE NCBI [Online] XP055238010 Database accession no. EFJ66882

<110> CJ Cheiljedang
<120> Microorganisms with enhanced intracellular ATP level and method for production of L-amino acids using the same
<130> PN103858
<160> 21
<170> KopatentIn 2.0
<210> 1
   <211> 798
   <212> DNA
   <213> Escherichia coli
<220>
   <221> gene
   <222> (1)..(798)
   <223> ORF of fhuC
<400> 1
   atgcaggaat acacgaatca ttccgatacc acttttgcac tgcgtaatat ctcctttcgt      60
   gtgcccgggc gcacgctttt gcatccgctg tcgttaacct ttcctgccgg gaaagtgacc     120
   ggtctgattg gtcacaacgg ttctggtaaa tccactctgc tcaaaatgct tggccgtcat     180
   cagccgccgt cggaagggga gattcttctt gatgcccaac cgctggaaag ctggagcagc     240
   aaagcgtttg cccgcaaagt ggcttatttg ccgcagcagc ttcctccggc agaagggatg     300
   accgtgcgtg aactggtggc gattggtcgt tacccgtggc atggcgcgct ggggcgcttt     360
   ggggcggcag atcgcgaaaa agtcgaggaa gctatctcgc tggttggctt aaaaccgctg     420
   gcgcatcggc tggtcgatag tctctctggc ggcgaacgtc agcgggcgtg gatcgccatg     480
   ctggtggcgc aggatagccg ttgtctgttg ctcgacgaac cgacctcggc gctggatatc     540
   gcccaccagg ttgatgtgct gtcgctggtg caccgtttaa gtcaggagcg tggcctgacg     600
   gtcattgccg tgttgcacga tatcaatatg gcggcacgct actgtgatta tctggtcgcc     660
   ctgcgcggcg gtgaaatgat tgctcaggga acgcctgcgg aaattatgcg cggcgaaacc     720
   ctcgaaatga tttatggcat cccgatgggt attttgccgc atccggcggg tgctgcacct     780
   gtgagttttg tttattga                                                   798
<210> 2
   <211> 891
   <212> DNA
   <213> Escherichia coli
<220>
   <221> gene
   <222> (1)..(891)
   <223> ORF of fhuD
<400> 2
   atgagcggct tacctcttat ttcgcgccgt cgactgttaa cggcgatggc gctttctccg     60
   ttgttatggc agatgaatac cgcccacgcg gcggctattg atcccaatcg tattgtggcg    120
   ctggagtggt tgccggtgga attactgctg gcgctcggca tcgtgcctta cggcgtggcg    180
   gataccatca actatcgcct gtgggtcagc gaaccaccat tgccggactc agtgatcgac    240
   gtcggtttgc gcacagaacc taaccttgaa ctgctgaccg aaatgaaacc atcgtttatg    300
   gtctggtcgg caggatatgg cccttcacca gaaatgctgg ctcgtattgc gccgggtcgc    360
   ggatttaact tcagtgacgg caaacagccg ttggcgatgg cgcgtaaatc gctgacggaa    420
   atggcagatt tacttaacct gcaaagcgca gcggaaacgc atttagcgca atatgaagac    480
   tttatccgca gcatgaaacc ccgctttgtg aagcgtggtg cgcgtccgtt attgctgacg    540
   acgcttatcg atccgcgcca tatgctggtc ttcggtccaa acagcttgtt ccaggaaatt    600
   cttgatgagt acggcatccc aaatgcctgg caaggggaaa ccaacttctg gggcagtacc    660
   gccgtcagta tcgatcgtct ggcggcgtat aaagacgttg atgtgctctg ttttgatcac    720
   gacaacagca aagacatgga tgcgctaatg gcaacgccgc tgtggcaggc catgccgttt    780
   gtccgcgccg gacgctttca gcgcgtacct gcagtctggt tttatggtgc gacgctctcg    840
   gcaatgcact ttgtgcgcgt tctggataac gccatcggag gtaaagcgtg a             891
<210> 3
   <211> 1983
   <212> DNA
   <213> Escherichia coli
<220>
   <221> gene
   <222> (1)..(1983)
   <223> ORF of fhuB
<400> 3
   gtgagtaaac gaattgcgct tttcccggcg ttattgctgg cgctgttagt gattgtcgct      60
   acggcgctca cctggatgaa cttctcgcag gcgctgccgc gtagccagtg ggcgcaggct     120
   gcctggtcgc cggatattga cgtcatcgag cagatgattt ttcactacag cttgttgccg     180
   cgtctggcga tttcgctgct ggtgggcgcg ggtctggggc tggtgggcgt gctgtttcag     240
   caagtgctgc gtaacccgct ggcggagccg acgacgcttg gcgttgctac aggcgcgcaa     300
   ctggggatta ccgtcactac gctctgggcg atccctggtg cgatggcgag ccagtttgct     360
   gcgcaggcag gggcttgtgt tgttggctta attgtctttg gcgtcgcgtg ggggaaacgg     420
   ctgtcgccgg taacgctgat tctcgcgggg ttggtagtga gcctttattg cggcgcaatc     480
   aatcagttac tggttatctt ccatcatgac caactgcaaa gcatgtttct gtggagcact     540
   ggaacgctga cgcaaaccga ctggggcggc gttgagcgtt tatggccgca gctgctgggc     600
   ggtgtgatgc tgacgttgct gctacttcgt ccgttaaccc tgatggggct tgatgatggc      660
   gtggcgcgca atctcgggct ggccttgtcg cttgcgcgtc tggcagcgct gtcgctggcg      720
   attgtcatca gtgcgctgct ggtgaacgct gtggggatta tcggctttat cgggttgttc      780
   gcgccgctgc tggcaaaaat gctgggggcg cggcgtctgc tgccacgact gatgctggcg      840
   tcgttgattg gtgcgctgat cctctggctt tccgatcaaa tcatcctctg gctgactcgc      900
   gtgtggatgg aagtgtccac cggttcggtc actgcgttga tcggtgcgcc gctgctactg      960
   tggctgttgc cgcgtttacg cagcattagc gcgccggata tgaaggtcaa cgatcgtgtc     1020
   gcggctgaac gccaacatgt gctggcgttt gccctcgcgg gcggcgtgct gctgttgatg     1080
   gctgtggtgg tggcgctgtc gtttggtcgt gatgcgcacg gctggacgtg ggcgagcggg     1140
   gcgttgctcg aggatttaat gccctggcgc tggccgcgaa ttatggcggc gctgtttgcg     1200
   ggcgtcatgc tggcggtggc gggctgtatt attcagcgac tgaccggaaa cccgatggca     1260
   agcccggaag tgctggggat tagctccggc gcggcgtttg gcgtggtgtt gatgctgttt     1320
   ctggtgccgg gtaatgcctt tggctggctg ttacctgcag gcagtctcgg cgcggcggtg     1380
   acgctgttga tcattatgat cgccgccggc cgcggtggat tttccccaca ccgtatgtta     1440
   ctggcgggga tggcgttaag caccgcgttc accatgcttt tgatgatgtt gcaggcaagt     1500
   ggtgacccgc gaatggcgca agtgctgacc tggatttccg gttcgaccta caacgcgacc     1560
   gatgcgcagg tctggcgcac cggaattgtg atggtgattt tgctggcgat taccccgctg     1620
   tgccgccgct ggctgaccat tttaccgctg ggtggtgata ccgcccgagc cgtaggaatg     1680
   gcgctgacgc cgacgcgaat tgcgctgctg ctgttagcgg cttgcctgac ggcgaccgcg     1740
   acgatgacta ttggaccgtt gagttttgtt ggtttaatgg caccgcatat tgcgcggatg     1800
   atgggctttc gacggacgat gccacacatc gtaatttcgg cgctggtggg tggtttactg     1860
   ctggtgttcg ctgactggtg tgggcggatg gtgctgtttc cattccagat cccggcgggg     1920
   ctgctgtcaa cctttatcgg cgcgccatat tttatctatt tgttgagaaa gcagagccgt     1980
   taa                                                                    1983
<210> 4
   <211> 3667
   <212> DNA
   <213> Escherichia coli
<220>
   <221> gene
   <222> (1)..(3667)
   <223> fhuCDB operon
<400> 4
   atgcaggaat acacgaatca ttccgatacc acttttgcac tgcgtaatat ctcctttcgt 60
   gtgcccgggc gcacgctttt gcatccgctg tcgttaacct ttcctgccgg gaaagtgacc     120
   ggtctgattg gtcacaacgg ttctggtaaa tccactctgc tcaaaatgct tggccgtcat     180
   cagccgccgt cggaagggga gattcttctt gatgcccaac cgctggaaag ctggagcagc     240
   aaagcgtttg cccgcaaagt ggcttatttg ccgcagcagc ttcctccggc agaagggatg     300
   accgtgcgtg aactggtggc gattggtcgt tacccgtggc atggcgcgct ggggcgcttt     360
   ggggcggcag atcgcgaaaa agtcgaggaa gctatctcgc tggttggctt aaaaccgctg     420
   gcgcatcggc tggtcgatag tctctctggc ggcgaacgtc agcgggcgtg gatcgccatg     480
   ctggtggcgc aggatagccg ttgtctgttg ctcgacgaac cgacctcggc gctggatatc     540
   gcccaccagg ttgatgtgct gtcgctggtg caccgtttaa gtcaggagcg tggcctgacg     600
   gtcattgccg tgttgcacga tatcaatatg gcggcacgct actgtgatta tctggtcgcc     660
   ctgcgcggcg gtgaaatgat tgctcaggga acgcctgcgg aaattatgcg cggcgaaacc     720
   ctcgaaatga tttatggcat cccgatgggt attttgccgc atccggcggg tgctgcacct     780
   gtgagttttg tttattgatg agcggcttac ctcttatttc gcgccgtcga ctgttaacgg     840
   cgatggcgct ttctccgttg ttatggcaga tgaataccgc ccacgcggcg gctattgatc     900
   ccaatcgtat tgtggcgctg gagtggttgc cggtggaatt actgctggcg ctcggcatcg     960
   tgccttacgg cgtggcggat accatcaact atcgcctgtg ggtcagcgaa ccaccattgc    1020
   cggactcagt gatcgacgtc ggtttgcgca cagaacctaa ccttgaactg ctgaccgaaa    1080
   tgaaaccatc gtttatggtc tggtcggcag gatatggccc ttcaccagaa atgctggctc    1140
   gtattgcgcc gggtcgcgga tttaacttca gtgacggcaa acagccgttg gcgatggcgc    1200
   gtaaatcgct gacggaaatg gcagatttac ttaacctgca aagcgcagcg gaaacgcatt    1260
   tagcgcaata tgaagacttt atccgcagca tgaaaccccg ctttgtgaag cgtggtgcgc    1320
   gtccgttatt gctgacgacg cttatcgatc cgcgccatat gctggtcttc ggtccaaaca    1380
   gcttgttcca ggaaattctt gatgagtacg gcatcccaaa tgcctggcaa ggggaaacca    1440
   acttctgggg cagtaccgcc gtcagtatcg atcgtctggc ggcgtataaa gacgttgatg    1500
   tgctctgttt tgatcacgac aacagcaaag acatggatgc gctaatggca acgccgctgt    1560
   ggcaggccat gccgtttgtc cgcgccggac gctttcagcg cgtacctgca gtctggtttt    1620
   atggtgcgac gctctcggca atgcactttg tgcgcgttct ggataacgcc atcggaggta    1680
   aagcgtgagt aaacgaattg cgcttttccc ggcgttattg ctggcgctgt tagtgattgt    1740
   cgctacggcg ctcacctgga tgaacttctc gcaggcgctg ccgcgtagcc agtgggcgca    1800
   ggctgcctgg tcgccggata ttgacgtcat cgagcagatg atttttcact acagcttgtt    1860
   gccgcgtctg gcgatttcgc tgctggtggg cgcgggtctg gggctggtgg gcgtgctgtt      1920
   tcagcaagtg ctgcgtaacc cgctggcgga gccgacgacg cttggcgttg ctacaggcgc 1980
   gcaactgggg attaccgtca ctacgctctg ggcgatccct ggtgcgatgg cgagccagtt 2040
   tgctgcgcag gcaggggctt gtgttgttgg cttaattgtc tttggcgtcg cgtgggggaa 2100
   acggctgtcg ccggtaacgc tgattctcgc ggggttggta gtgagccttt attgcggcgc 2160
   aatcaatcag ttactggtta tcttccatca tgaccaactg caaagcatgt ttctgtggag 2220
   cactggaacg ctgacgcaaa ccgactgggg cggcgttgag cgtttatggc cgcagctgct 2280
   gggcggtgtg atgctgacgt tgctgctact tcgtccgtta accctgatgg ggcttgatga 2340
   tggcgtggcg cgcaatctcg ggctggcctt gtcgcttgcg cgtctggcag cgctgtcgct 2400
   ggcgattgtc atcagtgcgc tgctggtgaa cgctgtgggg attatcggct ttatcgggtt 2460
   gttcgcgccg ctgctggcaa aaatgctggg ggcgcggcgt ctgctgccac gactgatgct 2520
   ggcgtcgttg attggtgcgc tgatcctctg gctttccgat caaatcatcc tctggctgac 2580
   tcgcgtgtgg atggaagtgt ccaccggttc ggtcactgcg ttgatcggtg cgccgctgct 2640
   actgtggctg ttgccgcgtt tacgcagcat tagcgcgccg gatatgaagg tcaacgatcg 2700
   tgtcgcggct gaacgccaac atgtgctggc gtttgccctc gcgggcggcg tgctgctgtt 2760
   gatggctgtg gtggtggcgc tgtcgtttgg tcgtgatgcg cacggctgga cgtgggcgag 2820
   cggggcgttg ctcgaggatt taatgccctg gcgctggccg cgaattatgg cggcgctgtt 2880
   tgcgggcgtc atgctggcgg tggcgggctg tattattcag cgactgaccg gaaacccgat 2940
   ggcaagcccg gaagtgctgg ggattagctc cggcgcggcg tttggcgtgg tgttgatgct 3000
   gtttctggtg ccgggtaatg cctttggctg gctgttacct gcaggcagtc tcggcgcggc 3060
   ggtgacgctg ttgatcatta tgatcgccgc cggccgcggt ggattttccc cacaccgtat 3120
   gttactggcg gggatggcgt taagcaccgc gttcaccatg cttttgatga tgttgcaggc 3180
   aagtggtgac ccgcgaatgg cgcaagtgct gacctggatt tccggttcga cctacaacgc 3240
   gaccgatgcg caggtctggc gcaccggaat tgtgatggtg attttgctgg cgattacccc 3300
   gctgtgccgc cgctggctga ccattttacc gctgggtggt gataccgccc gagccgtagg 3360
   aatggcgctg acgccgacgc gaattgcgct gctgctgtta gcggcttgcc tgacggcgac 3420
   cgcgacgatg actattggac cgttgagttt tgttggttta atggcaccgc atattgcgcg 3480
   gatgatgggc tttcgacgga cgatgccaca catcgtaatt tcggcgctgg tgggtggttt 3540
   actgctggtg ttcgctgact ggtgtgggcg gatggtgctg tttccattcc agatcccggc 3600
   ggggctgctg tcaaccttta tcggcgcgcc atattttatc tatttgttga gaaagcagag 3660
   ccgttaa 3667
<210> 5
   <211> 265
   <212> PRT
   <213> Escherichia coli
<220>
   <221> PEPTIDE
   <222> (1)..(265)
   <223> FhuC
<400> 5
   Met Gln Glu Tyr Thr Asn His Ser Asp Thr Thr Phe Ala Leu Arg Asn
   1               5                   10                  15
   Ile Ser Phe Arg Val Pro Gly Arg Thr Leu Leu His Pro Leu Ser Leu
               20                  25                  30
   Thr Phe Pro Ala Gly Lys Val Thr Gly Leu Ile Gly His Asn Gly Ser
           35                  40                  45
   Gly Lys Ser Thr Leu Leu Lys Met Leu Gly Arg His Gln Pro Pro Ser
       50                  55                  60
   Glu Gly Glu Ile Leu Leu Asp Ala Gln Pro Leu Glu Ser Trp Ser Ser
   65                  70                  75                  80
   Lys Ala Phe Ala Arg Lys Val Ala Tyr Leu Pro Gln Gln Leu Pro Pro
                   85                  90                  95
   Ala Glu Gly Met Thr Val Arg Glu Leu Val Ala Ile Gly Arg Tyr Pro
               100                 105                 110
   Trp His Gly Ala Leu Gly Arg Phe Gly Ala Ala Asp Arg Glu Lys Val
           115                 120                 125
   Glu Glu Ala Ile Ser Leu Val Gly Leu Lys Pro Leu Ala His Arg Leu
       130                 135                 140
   Val Asp Ser Leu Ser Gly Gly Glu Arg Gln Arg Ala Trp Ile Ala Met
   145                 150                 155                 160
   Leu Val Ala Gln Asp Ser Arg Cys Leu Leu Leu Asp Glu Pro Thr Ser
                   165                 170                 175
   Ala Leu Asp Ile Ala His Gln Val Asp Val Leu Ser Leu Val His Arg
               180                 185                 190
   Leu Ser Gln Glu Arg Gly Leu Thr Val Ile Ala Val Leu His Asp Ile
           195                 200                 205
   Asn Met Ala Ala Arg Tyr Cys Asp Tyr Leu Val Ala Leu Arg Gly Gly
       210                 215                 220
   Glu Met Ile Ala Gln Gly Thr Pro Ala Glu Ile Met Arg Gly Glu Thr
   225                 230                 235                 240
   Leu Glu Met Ile Tyr Gly Ile Pro Met Gly Ile Leu Pro His Pro Ala
                   245                 250                 255
   Gly Ala Ala Pro Val Ser Phe Val Tyr
               260                 265
<210> 6
   <211> 296
   <212> PRT
   <213> Escherichia coli
<220>
   <221> PEPTIDE
   <222> (1)..(296)
   <223> FhuD
<400> 6
   Met Ser Gly Leu Pro Leu Ile Ser Arg Arg Arg Leu Leu Thr Ala Met
   1               5                   10                  15
   Ala Leu Ser Pro Leu Leu Trp Gln Met Asn Thr Ala His Ala Ala Ala
               20                  25                  30
   Ile Asp Pro Asn Arg Ile Val Ala Leu Glu Trp Leu Pro Val Glu Leu
           35                  40                  45
   Leu Leu Ala Leu Gly Ile Val Pro Tyr Gly Val Ala Asp Thr Ile Asn
       50                  55                  60
   Tyr Arg Leu Trp Val Ser Glu Pro Pro Leu Pro Asp Ser Val Ile Asp
   65                  70                  75                  80
   Val Gly Leu Arg Thr Glu Pro Asn Leu Glu Leu Leu Thr Glu Met Lys
                   85                  90                  95
   Pro Ser Phe Met Val Trp Ser Ala Gly Tyr Gly Pro Ser Pro Glu Met
               100                 105                 110
   Leu Ala Arg Ile Ala Pro Gly Arg Gly Phe Asn Phe Ser Asp Gly Lys
           115                 120                 125
   Gln Pro Leu Ala Met Ala Arg Lys Ser Leu Thr Glu Met Ala Asp Leu
       130                 135                 140
   Leu Asn Leu Gln Ser Ala Ala Glu Thr His Leu Ala Gln Tyr Glu Asp
   145                 150                 155                 160
   Phe Ile Arg Ser Met Lys Pro Arg Phe Val Lys Arg Gly Ala Arg Pro
                   165                 170                 175
   Leu Leu Leu Thr Thr Leu Ile Asp Pro Arg His Met Leu Val Phe Gly
               180                 185                 190
   Pro Asn Ser Leu Phe Gln Glu Ile Leu Asp Glu Tyr Gly Ile Pro Asn
           195                 200                 205
   Ala Trp Gln Gly Glu Thr Asn Phe Trp Gly Ser Thr Ala Val Ser Ile
       210                 215                 220
   Asp Arg Leu Ala Ala Tyr Lys Asp Val Asp Val Leu Cys Phe Asp His
   225                 230                 235                 240
   Asp Asn Ser Lys Asp Met Asp Ala Leu Met Ala Thr Pro Leu Trp Gln
                   245                 250                 255
   Ala Met Pro Phe Val Arg Ala Gly Arg Phe Gln Arg Val Pro Ala Val
               260                 265                 270
   Trp Phe Tyr Gly Ala Thr Leu Ser Ala Met His Phe Val Arg Val Leu
           275                 280                 285
   Asp Asn Ala Ile Gly Gly Lys Ala
       290                 295
<210> 7
   <211> 660
   <212> PRT
   <213> Escherichia coli
<220>
   <221> PEPTIDE
   <222> (1)..(660)
   <223> FhuB
<400> 7
   Met Ser Lys Arg Ile Ala Leu Phe Pro Ala Leu Leu Leu Ala Leu Leu
   1               5                   10                  15
   Val Ile Val Ala Thr Ala Leu Thr Trp Met Asn Phe Ser Gln Ala Leu
               20                  25                  30
   Pro Arg Ser Gln Trp Ala Gln Ala Ala Trp Ser Pro Asp Ile Asp Val
           35                  40                  45
   Ile Glu Gln Met Ile Phe His Tyr Ser Leu Leu Pro Arg Leu Ala Ile
       50                  55                  60
   Ser Leu Leu Val Gly Ala Gly Leu Gly Leu Val Gly Val Leu Phe Gln
   65                  70                  75                  80
   Gln Val Leu Arg Asn Pro Leu Ala Glu Pro Thr Thr Leu Gly Val Ala
                   85                  90                  95
   Thr Gly Ala Gln Leu Gly Ile Thr Val Thr Thr Leu Trp Ala Ile Pro
               100                 105                 110
   Gly Ala Met Ala Ser Gln Phe Ala Ala Gln Ala Gly Ala Cys Val Val
           115                 120                 125
   Gly Leu Ile Val Phe Gly Val Ala Trp Gly Lys Arg Leu Ser Pro Val
       130                 135                 140
   Thr Leu Ile Leu Ala Gly Leu Val Val Ser Leu Tyr Cys Gly Ala Ile
   145                 150                 155                 160
   Asn Gln Leu Leu Val Ile Phe His His Asp Gln Leu Gln Ser Met Phe
                   165                 170                 175
   Leu Trp Ser Thr Gly Thr Leu Thr Gln Thr Asp Trp Gly Gly Val Glu
               180                 185                 190
   Arg Leu Trp Pro Gln Leu Leu Gly Gly Val Met Leu Thr Leu Leu Leu
           195                 200                 205
   Leu Arg Pro Leu Thr Leu Met Gly Leu Asp Asp Gly Val Ala Arg Asn
       210                 215                 220
   Leu Gly Leu Ala Leu Ser Leu Ala Arg Leu Ala Ala Leu Ser Leu Ala
   225                 230                 235                 240
   Ile Val Ile Ser Ala Leu Leu Val Asn Ala Val Gly Ile Ile Gly Phe
                   245                 250                 255
   Ile Gly Leu Phe Ala Pro Leu Leu Ala Lys Met Leu Gly Ala Arg Arg
               260                 265                 270
   Leu Leu Pro Arg Leu Met Leu Ala Ser Leu Ile Gly Ala Leu Ile Leu
           275                 280                 285
   Trp Leu Ser Asp Gln Ile Ile Leu Trp Leu Thr Arg Val Trp Met Glu
       290                 295                 300
   Val Ser Thr Gly Ser Val Thr Ala Leu Ile Gly Ala Pro Leu Leu Leu
   305                 310                 315                 320
   Trp Leu Leu Pro Arg Leu Arg Ser Ile Ser Ala Pro Asp Met Lys Val
                   325                 330                 335
   Asn Asp Arg Val Ala Ala Glu Arg Gln His Val Leu Ala Phe Ala Leu
               340                 345                 350
   Ala Gly Gly Val Leu Leu Leu Met Ala Val Val Val Ala Leu Ser Phe
           355                 360                 365
   Gly Arg Asp Ala His Gly Trp Thr Trp Ala Ser Gly Ala Leu Leu Glu
       370                 375                 380
   Asp Leu Met Pro Trp Arg Trp Pro Arg Ile Met Ala Ala Leu Phe Ala
   385                 390                 395                 400
   Gly Val Met Leu Ala Val Ala Gly Cys Ile Ile Gln Arg Leu Thr Gly
                   405                 410                 415
   Asn Pro Met Ala Ser Pro Glu Val Leu Gly Ile Ser Ser Gly Ala Ala
               420                 425                 430
   Phe Gly Val Val Leu Met Leu Phe Leu Val Pro Gly Asn Ala Phe Gly
           435                 440                 445
   Trp Leu Leu Pro Ala Gly Ser Leu Gly Ala Ala Val Thr Leu Leu Ile
       450                 455                 460
   Ile Met Ile Ala Ala Gly Arg Gly Gly Phe Ser Pro His Arg Met Leu
   465                 470                 475                 480
   Leu Ala Gly Met Ala Leu Ser Thr Ala Phe Thr Met Leu Leu Met Met
                   485                 490                 495
   Leu Gln Ala Ser Gly Asp Pro Arg Met Ala Gln Val Leu Thr Trp Ile
               500                 505                 510
   Ser Gly Ser Thr Tyr Asn Ala Thr Asp Ala Gln Val Trp Arg Thr Gly
           515                 520                 525
   Ile Val Met Val Ile Leu Leu Ala Ile Thr Pro Leu Cys Arg Arg Trp
       530                 535                 540
   Leu Thr Ile Leu Pro Leu Gly Gly Asp Thr Ala Arg Ala Val Gly Met
   545                 550                 555                 560
   Ala Leu Thr Pro Thr Arg Ile Ala Leu Leu Leu Leu Ala Ala Cys Leu
                   565                 570                 575
   Thr Ala Thr Ala Thr Met Thr Ile Gly Pro Leu Ser Phe Val Gly Leu
   580 585 590
   Met Ala Pro His Ile Ala Arg Met Met Gly Phe Arg Arg Thr Met Pro
   595 600 605
   His Ile Val Ile Ser Ala Leu Val Gly Gly Leu Leu Leu Val Phe Ala
   610 615 620
   Asp Trp Cys Gly Arg Met Val Leu Phe Pro Phe Gln Ile Pro Ala Gly
   625 630 635 640
   Leu Leu Ser Thr Phe Ile Gly Ala Pro Tyr Phe Ile Tyr Leu Leu Arg
   645 650 655
   Lys Gln Ser Arg
   660
<210> 8
   <211> 70
   <212> DNA
   <213> Artificial Sequence
<220>
   <223> Primer 1
<400> 8
   ctcctttcgt gtgcccgggc gcacgctttt gcatccgctg tcgttaacct aggtgacact 60
   atagaacgcg 70
<210> 9
   <211> 70
   <212> DNA
   <213> Artificial Sequence
<220>
   <223> Primer 2
<400> 9
   caataaacaa aactcacagg tgcagcaccc gccggatgcg gcaaaatacc tagtggatct 60
   gatgggtacc 70
<210> 10
   <211> 70
   <212> DNA
   <213> Artificial Sequence
<220>
   <223> Primer 3
<400> 10
   cgactgttaa cggcgatggc gctttctccg ttgttatggc agatgaatac aggtgacact 60
   atagaacgcg 70
<210> 11
   <211> 70
   <212> DNA
   <213> Artificial Sequence
<220>
   <223> Primer 4
<400> 11
   tcacgcttta cctccgatgg cgttatccag aacgcgcaca aagtgcattg tagtggatct 60
   gatgggtacc 70
<210> 12
   <211> 70
   <212> DNA
   <213> Artificial Sequence
<220>
   <223> Primer 5
<400> 12
   gtgagtaaac gaattgcgct tttcccggcg ttattgctgg cgctgttagt aggtgacact 60
   atagaacgcg 70
<210> 13
   <211> 70
   <212> DNA
   <213> Artificial Sequence
<220>
   <223> Primer 6
<400> 13
   aggttgacag cagccccgcc gggatctgga atggaaacag caccatccgc tagtggatct 60
   gatgggtacc 70
<210> 14
   <211> 70
   <212> DNA
   <213> Artificial Sequence
<220>
   <223> Primer 7
<400> 14
   atgcaggaat acacgaatca ttccgatacc acttttgcac tgcgtaatat ctcctttcgt 60
   gtgcccgggc 70
<210> 15
   <211> 70
   <212> DNA
   <213> Artificial Sequence
<220>
   <223> Primer 8
<400> 15
   gccatcgccg ttaacagtcg acggcgcgaa ataagaggta agccgctcat caataaacaa 60
   aactcacagg 70
<210> 16
   <211> 70
   <212> DNA
   <213> Artificial Sequence
<220>
   <223> Primer 9
<400> 16
   cctgtgagtt ttgtttattg atgagcggct tacctcttat ttcgcgccgt cgactgttaa 60
   cggcgatggc 70
<210> 17
   <211> 70
   <212> DNA
   <213> Artificial Sequence
<220>
   <223> Primer 10
<400> 17
   aatcactaac agcgccagca ataacgccgg gaaaagcgca attcgtttac tcacgcttta 60
   cctccgatgg 70
<210> 18
   <211> 70
   <212> DNA
   <213> Artificial Sequence
<220>
   <223> Primer 11
<400> 18
   tcggcaatgc actttgtgcg cgttctggat aacgccatcg gaggtaaagc gtgagtaaac    60
   gaattgcgct                                                            70
<210> 19
   <211> 70
   <212> DNA
   <213> Artificial Sequence
<220>
   <223> Primer 12
<400> 19
   ttaacggctc tgctttctca acaaatagat aaaatatggc gcgccgataa aggttgacag    60
   cagccccgcc                                                            70
<210> 20
   <211> 26
   <212> DNA
   <213> Artificial Sequence
<220>
   <223> Primer 13
<400> 20
   gaatacgtcg ccagctgctt taacac 26
<210> 21
   <211> 24
   <212> DNA
   <213> Artificial Sequence
<220>
   <223> Primer 14
<400> 21
   tggtttgtcg gatgcggcgt gaac 24

## Description

### TECHNICAL FIELD

The present application relates to a recombinant microorganism having an improved intracellular energy level and a method for producing L-amino acids using the microorganism.

### BACKGROUND ART

For production of a desired material using a microorganism, desired material-specific approaches have been mainly used, such as enhancement of expression of genes encoding enzymes involved in the production of the desired material or removal of unnecessary genes. For example, a number of useful strains including *E. coli* capable of producing a desired L-amino acid with a high yield have been developed by enhancement of a biosynthetic pathway of the L-amino acid. High-yield production of useful desired materials using microorganisms requires production and maintenance of sufficient energy.

For *In vivo* biosynthesis of materials such as proteins, nucleic acids, etc., energy conserved in the form of NADH, NADPH and ATP (Adenosine-5'-triphosphate) is used. Particularly, ATP is an energy carrier that transports chemical energy produced in metabolic reactions to various activities of organisms.

ATP is mainly produced in metabolic processes of microorganisms. Major intracellular ATP production pathways are substrate level phosphorylation that takes place via glycolysis or oxidative phosphorylation that produces ATP through the electron transport system using a reducing power accumulated in NADH, etc. via glycolysis. The generated ATP is consumed *in vivo* activities such as biosynthesis, motion, signal transduction, and cell division. Therefore, industrial microorganisms used for the production of useful desired materials generally exhibit high ATP demand. Accordingly, studies have been conducted to improve productivity by increasing intracellular energy levels upon mass-production of useful desired materials (Biotechnol Adv (2009) 27:94-101).

Iron is one of elements essential for maintenance of homeostasis of microorganisms, and *E. coli* utilizes various routes for uptake of iron (Mol Microbiol (2006) 62:120-131). One of the iron uptake routes is to uptake iron via FhuCDB complex channels formed by FhuC, FhuD, and FhuB proteins. Recently, it was revealed that in the presence of excess L-tryptophan in cells, TrpR protein regulating expression of genes involved in L-tryptophan biosynthesis forms a complex with L-tryptophan, and in turn, this complex binds to a regulatory region of fhuCDB operon, suggesting the possibility of a correlation between iron uptake via the FhuCDB protein complex and L-tryptophan biosynthesis. However, function of the FhuCDB protein complex in L-tryptophan biosynthesis, and its effect on iron uptake have not been clarified yet (Nat Chem Biol (2012) 8:65-71).

EP 1 829 965 relates to an *E. coli* mutant strain having chromosomal DNA that is at least 470 kbp shorter than that of a wild-type *E. coli* strain, and that exhibits the property that the number of the cells after a certain period in culture is greater than a wild-type strain.

WO 2007/024756 relates to a reduced genome strains of *E. coli* MGl 655. WO 2007/024756 mentions strains that have one or more of an equal or improved growth rate, transformation efficiency, protein expression, DNA production, DNA yield and/or DNA quality compared to the parental strain and commercially available strains.

Mademidis et al., 1998, Molecular and General Genetics, 258 (12): 156-165 relates to the transport activity of FhuA, FhuC, FhuD and FhuB derivatives in a system free of polar effects, and stoichiometry of components involved in ferrichrome uptake.

WO 02/077183 relates to sequences of antisense nucleic acids which inhibit the proliferation of prokaryotes.

The present inventors have studied methods of improving ATP levels and increasing producibility of useful desired materials such as L-amino acids, and they found that intracellular ATP levels may be improved by inactivation of the function of the FhuCDB protein complex by deletion of fhuCDB gene, and as a result, producibility of desired materials may be increased, thereby completing the present application.

### DETAILED DESCRIPTION OF THE INVENTION

### TECHNICAL PROBLEM

An object of the present application is to provide a microorganism having an improved intracellular ATP level.

Another object of the present application is to provide a method for producing a desired material using the microorganism having the improved intracellular ATP level.

### TECHNICAL SOLUTION

The invention is as defined in the accompanying claims.

The present invention provides a method for producing L-amino acids, the method comprising:
culturing a microorganism of the genus *Escherichia* in a media, and
recovering L-amino acids from the culture media or the microorganism;
wherein the microorganism has an increased intracellular ATP level, compared to an unmodified strain, wherein one or more proteins selected from an amino acid sequence of SEQ ID NO: 5, an amino acid sequence of SEQ ID NO: 6, and an amino acid sequence of SEQ ID NO: 7, which constitute an iron uptake system, are inactivated in the microorganism;
wherein the microorganism of the genus *Escherichia* has an improved ability to produce L-amino acid, compared to an unmodified strain; and
wherein the L-amino acid is L-threonine or L-tryptophan.

The microorganism is a microorganism in which activities of one or more of iron uptake system-constituting protein FhuC, protein FhuD, and protein FhuB are inactivated, and therefore, has an increased intracellular ATP level, compared to an unmodified strain.

The term "FhuCDB", as used herein, is a component of an iron uptake system (fhu system) which includes expression products of fhuA, fhuC, fhuD and fhuB arranged in one operon. The fhuA encodes multi-functional OMP FhuA (79 kDa) which acts as a receptor for ferrichrome-iron, phages, bacterial toxins, and antibiotics. FhuA is specific to Fe³⁺-ferrichrome, and acts as a ligand-specific gated channel (Protein Sci 7, 1636-1638). The other proteins of the fhu system, namely, FhuD, FhuC and FhuB are also essential for the functions of the iron uptake system. A periplasmic protein, FhuD and cytoplasmic membrane-associated proteins, FhuC and FhuB form a FhuCDB complex, which functions to transport ferrichrome and other Fe³⁺-hydroxamate compounds (Fe³⁺-aerobactin, Fe³⁺-coprogen) across the cytoplasmic membrane from the periplasm into the cytoplasm (J Bacteriol 169, 3844-3849). Uptake of iron via the FhuCDB complex consumes one molecule of ATP, and for this iron uptake process, a protein complex, TonB-ExbB-ExbD provides energy (FEBS Lett 274, 85-88).

FhuC encodes a cytoplasmic membrane-associated protein of 29 kDa, and forms a channel for iron uptake, together with FhuD and FhuB. FhuC may have an amino acid sequence of SEQ ID NO: 5, and specifically, FhuC may be encoded by a nucleotide sequence of SEQ ID NO: 1.

FhuD encodes a cytoplasmic membrane-associated protein of 31 kDa, and forms a channel for iron uptake, together with FhuC and FhuB. FhuD may have an amino acid sequence of SEQ ID NO: 6, and specifically, FhuD may be encoded by a nucleotide sequence of SEQ ID NO: 2.

FhuB encodes a cytoplasmic membrane-associated protein of 41 kDa, and forms a channel for iron uptake, together with FhuC and FhuB. FhuB may have an amino acid sequence of SEQ ID NO: 7, and specifically, FhuB may be encoded by a nucleotide sequence of SEQ ID NO: 3.

Specifically, although proteins have identical activities, there are small differences in the amino acid sequences between subjects. Therefore, FhuC, FhuD, and FhuB may have SEQ ID NOs: 5, 6, and 7, respectively. Further, in the nucleotide sequences, various modifications may be made in the coding region provided that they do not change the amino acid sequences of the proteins expressed from the coding region, due to codon degeneracy or in consideration of the codons preferred by the organism in which they are to be expressed. The above-described nucleotide sequence is provided only as an example of various nucleotide sequences made by a method well known to those skilled in the art, but is not limited thereto.

The term "homology", as used herein, refers to a degree of identity between bases or amino acid residues after both sequences are aligned so as to best match in certain comparable regions in an amino acid or nucleotide sequence of a gene encoding a protein. If the homology is sufficiently high, expression products of the corresponding genes may have identical or similar activity. The percentage of the sequence identity may be determined using a known sequence comparison program, for example, BLASTN (NCBI), CLC Main Workbench (CLC bio), MegAlign™ (DNASTAR Inc), etc.

The term "microorganism", as used herein, refers to a microorganism having the improved intracellular ATP level, and which is of the genus *Escherichia.* Specifically, the microorganism may be *E. coli.*

The "unmodified strain", as used herein, refers to a microorganism which is not modified by a molecular biological technique such as mutation or recombination. Specifically, the unmodified strain refers to a microorganism before increasing the intracellular ATP level, in which the intracellular ATP level is increased by inactivating one or more of FhuC, FhuD, and FhuB constituting the iron uptake system, FhuCDB complex, thereby having a reduction of intracellular ATP consumption. That is, the unmodified strain refers to an original microorganism from which the recombinant microorganism is derived.

The microorganism includes inactivation of one or more of FhuC, FhuD, and FhuB, and specifically inactivation of a combination of FhuC, FhuD, and FhuB, and more specifically, inactivation of all of FhuC, FhuD, and FhuB.

The term "inactivation", as used herein, means that the activity of the corresponding protein is eliminated or weakened by mutation due to deletion, substitution, or insertion of part or all of the gene encoding the corresponding protein, by modification of an expression regulatory sequence to reduce the expression of the gene, by modification of the chromosomal gene sequence to weaken or eliminate the activity of the protein, or by combinations thereof.

Specifically, deletion of part or all of the gene encoding the protein may be performed by replacing a polynucleotide which encodes an endogenous target protein in the chromosome, with either a polynucleotide of which a partial sequence is deleted or a marker gene through a bacterial chromosome insertion vector. Further, a mutation may be induced using a mutagen such as chemicals or UV light, thereby obtaining a mutant having deletion of the corresponding gene, but is not limited thereto.

The term "expression regulatory sequence", as used herein, a nucleotide sequence regulating a gene expression, refers to a segment capable of increasing or decreasing expression of a particular gene in a subject, and may include a promoter, a transcription factor binding site, a ribosome-binding site, a sequence regulating the termination of transcription and translation, but is not limited thereto.

Specifically, modification of the expression regulatory sequence for causing a decrease in gene expression may be performed by inducing mutations in the expression regulatory sequence through deletion, insertion, conservative or non-conservative substitution of nucleotide sequence or a combination thereof to further weaken the activity of the expression regulatory sequence, or by replacing the expression regulatory sequence with of the sequence having weaker activity, but is not limited thereto.

The microorganism is a microorganism of the genus *Escherichia* having an improved producibility of the L-amino acids L-threonine or L-tryptophan, compared to an unmodified strain. In the microorganism of the genus *Escherichia* of the present application, one or more of proteins constituting FhuCDB complex are inactivated to inactivate the iron uptake pathway, and therefore, iron uptake via this pathway reduces ATP consumption. As a result, the microorganism of the genus *Escherichia* has an improved intracellular ATP level, compared to the unmodified strain, and consequently, the microorganism has the improved producibility of the L-amino acids L-threonine or L-tryptophan.

The term "microorganism having the improved producibility" refers to a microorganism having an improved producibility of the L-amino acids L-threonine or L-tryptophan, compared to an unmodified strain or a parent cell before modification.

In a specific embodiment, the microorganism may be *E. coli* having an improved producibility of L-tryptophan, wherein one or more of FhuC, FhuD, and FhuB from *E. coli* having a producibility of L-tryptophan were inactivated, and having improved intracellular ATP level, compared to an unmodified strain. The *E. coli* having the producibility of L-tryptophan may be obtained by increasing expression of an L-tryptophan operon gene, removing feedback inhibition by a final product L-tryptophan, or removing inhibition and attenuation of the L-tryptophan operon gene at a transcriptional level, but is not limited thereto.

In a specific embodiment of the present application, the microorganism may be *E. coli* having an improved producibility of L-threonine, wherein one or more of FhuC, FhuD, and FhuB from *E. coli* having a producibility of L-threonine were inactivated, and having improved intracellular ATP level, compared to an unmodified strain. The *E. coli* having the producibility of L-threonine may be obtained by increasing expression of an L-threonine operon gene, removing feedback inhibition by a final product L-threonine, or removing inhibition and attenuation of the L-threonine operon gene at a transcriptional level, but is not limited thereto.

In the method for producing L-amino acids according to a specific embodiment of the present application, the culturing of the microorganism having the producibility of L-amino acids may be performed according to an appropriate medium and culture conditions known in the art. The culturing procedures may be readily adjusted by those skilled in the art according to the selected microorganism. Examples of the culturing procedures include batch type, continuous type and fed-batch type, but are not limited thereto.

A medium used for the culturing must meet the requirements for the culturing of a specific microorganism. The culture media for various microorganisms are described in a literature ("Manual of Methods for General Bacteriology" by the American Society for Bacteriology, Washington D.C., USA, 1981.). These media include a variety of carbon sources, nitrogen sources, and trace elements. The carbon source includes carbohydrates such as glucose, lactose, sucrose, fructose, maltose, starch and cellulose; lipids such as soybean oil, sunflower oil, castor oil and coconut oil; fatty acids such as palmitic acid, stearic acid, and linoleic acid; alcohols such as glycerol and ethanol; and organic acids such as acetic acid. These carbon sources may be used alone or in combination, but are not limited thereto. The nitrogen source includes organic nitrogen sources, such as peptone, yeast extract, gravy, malt extract, corn steep liquor (CSL) and bean flour, and inorganic nitrogen sources such as urea, ammonium sulfate, ammonium chloride, ammonium phosphate, ammonium carbonate and ammonium nitrate. These nitrogen sources may be used alone or in combination, but are not limited thereto. Additionally, the medium may include potassium dihydrogen phosphate, dipotassium hydrogen phosphate, and corresponding sodium-containing salts thereof as a phosphorus source, but is not limited thereto. Also, the medium may include a metal such as magnesium sulfate or iron sulfate. In addition, amino acids, vitamins and proper precursors may be added as well.

Further, to maintain the culture under aerobic conditions, oxygen or oxygen-containing gas (e.g., air) may be injected into the culture. A temperature of the culture may be generally 20°C-45°C, and specifically 25°C-40°C. The culturing may be continued until production of L-amino acids such as L-threonine or L-tryptophan reaches a desired level, and specifically, a culturing time may be 10 hrs-100 hrs.

The method for producing L-amino acids according to the present application includes recovering of the L-amino acids L-threonine or L-tryptophan from the culture media or the microorganism thus obtained. Recovering of L-amino acids may be performed by a proper method known in the art, depending on the method of culturing the microorganism of the present application, for example, batch type, continuous type or fed-batch type, so as to purify or recover the desired L-amino acids from the culture of the microorganism, but is not limited thereto.

### ADVANTAGEOUS EFFECTS OF THE INVENTION

According to the present application, when a microorganism of the genus *Escherichia* having an improved intracellular ATP level, compared to an unmodified strain, and a method for producing a desired material using the same are used, the high intracellular ATP level enhances gene expression, biosynthesis, transport of materials, etc., thereby efficiently producing the useful desired material including proteins, L-amino acids, etc.

### DESCRIPTION OF THE DRAWINGS

FIG. 1 shows intracellular ATP levels of *E. coli* according to a specific embodiment of the present application, compared to an unmodified strain;
FIG. 2 shows intracellular ATP levels of wild-type-derived *E. coli* having a producibility of L-tryptophan according to a specific embodiment of the present application, compared to an unmodified strain;
FIG. 3 shows intracellular ATP levels of *E. coli* having a producibility of L-threonine according to a specific embodiment of the present application, compared to an unmodified strain;
FIG. 4 shows intracellular ATP levels of *E. coli* having a producibility of L-tryptophan according to a specific embodiment of the present application, compared to an unmodified strain;
FIG. 5 shows L-threonine producibility of *E. coli* having a producibility of L-threonine according to a specific embodiment of the present application, compared to an unmodified strain; and
FIG. 6 shows L-tryptophan producibility of *E. coli* having a producibility of L-tryptophan according to a specific embodiment of the present application, compared to an unmodified strain.

### MODE OF THE INVENTION

Hereinafter, the present application will be described in more detail with reference to Examples. However, these Examples are for illustrative purposes only, and the scope of the present application is not intended to be limited by these Examples.

### Example 1: Preparation of Wild-Type E. coli W3110 Having Inactivation of Proteins Encoded by fhuC. fhuD, and fhuB Genes

In this Example, *fhuC, fhuD,* and *fhuB* genes of wild-type *E. coli* W3110 (ATCC® 39936™) were deleted by homologous recombination, respectively.

The *fhuC, fhuD,* and *fhuB* genes to be deleted have nucleotide sequences of SEQ ID NOs: 1, 2, and 3, respectively and these genes exist in the form of operon of SEQ ID NO: 4.

To delete *fhuC, fhuD,* and *fhuB,* one-step inactivation using lambda Red recombinase developed by Datsenko KA, et al. was performed (Proc Natl Acad Sci USA., (2000) 97:6640-6645). As a marker to confirm the insertion into the gene, a chloramphenicol gene of pUCprmfmloxC, which was prepared by ligating an rmf promoter to pUC19 (New England Biolabs (USA)) and ligating a mutated loxP-CmR-loxP cassette obtained from pACYC184 (New England Biolab) thereto, was used (Korean Patent Application NO. 2009-0075549).

First, primary polymerase chain reaction (hereinbelow, referred to as 'PCR') was performed using pUCprmfmloxC as a template and primer combinations of SEQ ID NOs: 8 and 9, 10 and 11, 12 and 13, and 8 and 13 having a part of the *fhuC* and *fhuB* genes and a partial sequence of the chloramphenicol resistant gene of the pUCprmfmloxC gene under the conditions of 30 cycles of denaturation at 94°C for 30 seconds, annealing at 55°C for 30 seconds and elongation at 72°C for 1 minute, thereby obtaining PCR products of about 1.2 kb, ΔfhuC1st, ΔfhuD1st, ΔfhuB1st, and ΔfhuCDB1st.

Thereafter, the PCR products of 1.2 kb, ΔfhuC1st, ΔfhuD1st, ΔfhuB1st, and ΔfhuCDB1st obtained by PCR were electrophoresed on a 0.8% agarose gel, and then eluted and used as a template for secondary PCR. The secondary PCR was performed using the eluted primary PCR products as templates and the primer combinations of SEQ ID NOs: 14 and 15, 16 and 17, 18 and 19, 14 and 19 containing nucleotide sequences of 20 bp of the 5' and 3' regions of the PCR products obtained in the primary PCR under the conditions of 30 cycles of denaturation at 94°C for 30 seconds, annealing at 55°C for 30 seconds and elongation at 72°C for 1 minute, thereby obtaining PCR products of about 1.3 kb, ΔfhuC, ΔfhuD, ΔfhuB, and ΔfhuCDB. The PCR products thus obtained was electrophoresed on a 0.8% agarose gel, and then eluted, and used in recombination.

*E. coli* W3110, which was transformed with a pKD46 vector according to the one-step inactivation method developed by Datsenko KA et al. (Proc Natl Acad Sci USA., (2000) 97:6640-6645), was prepared as a competent strain, and transformation was performed by introducing the gene fragment of 1.3 kb obtained by primary and secondary PCR. The strains were cultured on the LB medium supplemented with chloramphenicol and transformants having chloramphenicol resistance were selected. Deletion of any or all of *fhuC, fhuD,* and *fhuB* was confirmed by PCR products of about 4.4 kb, about 4.3 kb, about 3.3 kb, and about 1.6 kb which were amplified by PCR using genomes obtained from the selected strains as templates and primers of SEQ ID NOs: 20 and 21.

After removal of pKD46 from the primary recombinant strains having chloramphenicol resistance thus obtained, a pJW168 vector (Gene, (2000) 247,255-264) was introduced into the primary recombinant strains having chloramphenicol resistance so as to remove the chloramphenicol marker gene from the strains (Gene, (2000) 247, 255-264). PCR was performed using primers of SEQ ID NOs: 20 and 21 to obtain PCR products of about 3.4 kb, about 3.3 kb, about 2.2 kb, and about 0.6 kb, indicating that the strains finally obtained had deletion of any or all of *fhuC,fhuD,* and *fhuB* genes. The strains were designated as *E. coli* W3110_ΔfhuC, W3110_ΔfhuD, W3110_ΔfhuB and W3110_ΔfhuCDB, respectively.

### Example 2: Measurement of Intracellular ATP Levels in Wild-Type E. coli-Derived fhuC. fhuD, and fhuB gene-deleted E. coli

In this Example, the intracellular ATP levels in the strains prepared in Example 1 were practically measured.

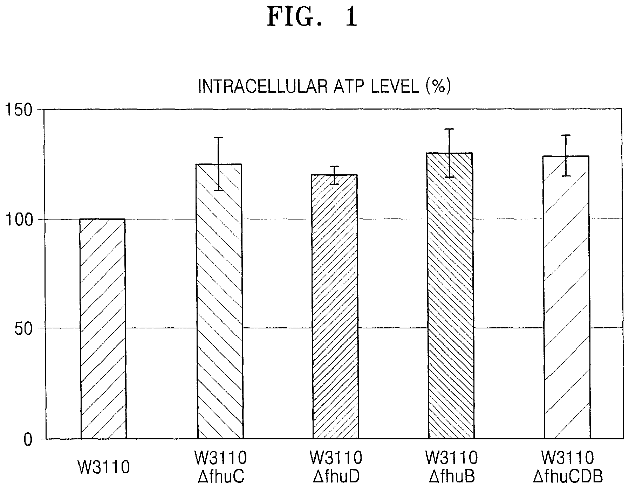

For this purpose, "An Efficient Method for Quantitative determination of Cellular ATP Synthetic Activity" developed by Kiyotaka Y. Hara et al., in which luciferase is used, was employed (J Biom Scre, (2006) Vol. 11, No.3, pp310-17). Briefly, *E. coli* W3110 which is an unmodified strain used in Example 1 and *E. coli* W3110_ΔfhuCDB obtained by gene deletion were cultured overnight in LB liquid medium containing glucose, respectively. After culturing, supernatants were removed by centrifugation, the cells thus obtained were washed with 100 mM Tris-Cl (pH 7.5), and then treated with PB buffer (permeable buffer: 40% [v/v] glucose, 0.8% [v/v] Triton X-100) for 30 minutes to release intracellular ATP from the cells. Next, supernatants were removed by centrifugation, and luciferin as a substrate for luciferase was added to the cells. The cells were allowed to react for 10 minutes. Color development by luciferase was measured using a luminometer to quantitatively determine ATP levels. The results are given in FIG. 1. The results of FIG. 1 were recorded as the average of three repeated experiments.

As shown in FIG. 1, the intracellular ATP levels in *E. coli* W3110_ΔfhuC, W3110_ΔfhuD, W3110_ΔfhuB and W3110_ΔfhuCDB prepared in Example 1, in which any or all of wild-type *E.* coli-derived *fhuC, fhuD,* and *fhuB* was/were deleted, were increased, compared to the unmodified strain, *E. coli* W3110.

### Example 3: Preparation of Wild-Type-Derived L-Tryptophan-Producinh Strain Having Inactivation of Proteins Encoded by fhuC, fhuD, and fhuB Genes and Measurement of Intracellular ATP Levels

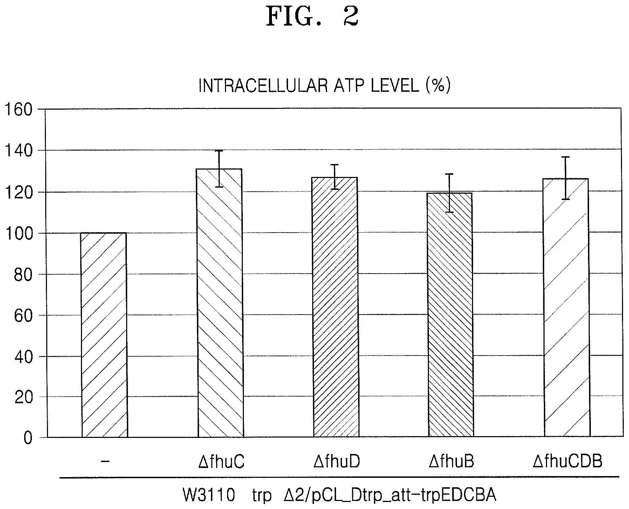

In this Example, any or all of *fhuC, fhuD,* and *fhuB* genes of a wild-type-derived L-tryptophan-producing strain, *E. coli* W3110 trpΔ2/pCL-Dtrp_att-trpEDCBA (Korean Patent Publication No. 10-2013-0082121) was/were deleted by homologous recombination as in Example 1 to prepare W3110 trpΔ2_ΔfhuC/pCL-Dtrp_att-trpEDCBA, W3110 trpΔ2_ΔfhuD/pCL-Dtrp_att-trpEDCBA, W3110 trpΔ2_ΔfhuB/pCL-Dtrp_att-trpEDCBA, and W3110 trpΔ2_ΔfhuCDB/pCL-Dtrp_att-trpEDCBA strains. In these strains thus prepared, intracellular ATP levels were measured in the same manner as in Example 2 and the results are given in FIG. 2.

As shown in FIG. 2, the intracellular ATP levels in the strains, which were prepared by deletion of any or all of *fhuC, fhuD,* and *fhuB* genes in the wild-type L-tryptophan-producing strain, were increased, compared to an unmodified strain and a control strain.

### Example 4: Examination of Titer of Wild-Type-Derived L-Tryptophan-Producinh Strain Having Inactivation of Proteins Encoded by fhuC, fhuD, and thub Genes

As described in Example 3, the wild-type-derived L-tryptophan-producing strain, W3110 trpΔ2/pCL-Dtrp_att-trpEDCBA and the strains with improved intracellular ATP levels prepared by deletion of any or all of *fhuC, fhuD,* and *fhuB* genes were subjected to titration using glucose as a carbon source.

Each of the strains was inoculated by a platinum loop on an LB solid medium, and cultured in an incubator at 37°C overnight, and then inoculated by a platinum loop into 25 mL of a glucose-containing titration medium containing a composition of Table 1. Then, the strains were cultured in an incubator at 37°C and at 200 rpm for 48 hours. The results are given in Table 2. All the results were recorded as the average of three repeated experiments.

**[Table 1]**

| Composition | Concentration (per liter) |
|---|---|
| Glucose | 60 g |
| K₂HPO₄ | 1 g |
| (NH₄)₂SO₄ | 15 g |
| NaCl | 1 g |
| MgSO₄·H₂O | 1 g |
| Sodium citrate | 5 g |
| Yeast extract | 2 g |
| CaCO₃ | 40 g |
| L-Phenylalanine | 0.15 g |
| L-tyrosine | 0.1 g |
| pH | 6.8 |

**[Table 2]**

| Strain | Production amount of L-tryptophan (mg/L)* |
|---|---|
| W3110 trpΔ2/pCL-Dtrp_att-trpEDCBA | 562 |
| W3110 trpΔ2_ΔfhuC/pCL-Dtrp_att-trpEDCBA | 781 |
| W3110 trpΔ2_ΔfhuD/pCL-Dtrp_att-trpEDCBA | 816 |
| W3110 trpΔ2_ΔfhuB/pCL-Dtrp_att-trpEDCBA | 779 |
| W3110 trpΔ2_ΔhuCDB/pCL-Dtrp_att-trpEDCBA | 796 |

| | |
|---|---|
| * measured at 48 hours | |

As shown in Table 2, it was demonstrated that the strains with improved intracellular ATP levels, prepared in Example 3 by deleting any or all of *fhuC, fhuD,* and *fhuB* genes in the wild-type L-tryptophan-producing strain W3110 trpΔ2/pCL-Dtrp_att-trpEDCBA, increased L-tryptophan production up to about 63%, compared to the unmodified strain trpΔ2/pCL-Dtrp_att-trpEDCBA. In view of the intracellular ATP levels confirmed in FIG. 2, these results indicate that L-tryptophan producibilities of the strains were increased by the increased intracellular ATP levels.

### Example 5: Preparation of L-Threonine-Producing Strain and L-Tryptophan Producing Strain Having Inactivation of Proteins Encoded by fhuC, fhuD, and fhuB Genes

In this Example, *fhuC, fhuD,* and *fhuB* genes of the L-tryptophan producing strain KCCM10812P (Korean Patent No. 0792095) and the L-threonine producing strain KCCM10541 (Korean Patent No. 0576342) were deleted by homologous recombination, respectively, as in Example 1.

The unmodified strain having L-tryptophan producibility, *E. coli* KCCM10812P is a strain derived from an *E. coli* variant (KFCC 10066) having L-phenylalanine producibility, and is a recombinant *E. coli* strain having L-tryptophan producibility, characterized in that chromosomal tryptophan auxotrophy was desensitized or removed, *pheA, trpR, mtr* and *tnaAB* genes were attenuated, and *aroG* and *trpE* genes were modified.

Also, the unmodified strain having L-threonine producibility, *E. coli* KCCM10541P is a strain derived from *E. coli* KFCC10718 (Korean Patent Publication No. 1992-0008365), and is *E. coli* having resistance to L-methionine analogue, a methionine auxotroph phenotype, resistance to L-threonine analogue, a leaky isoleucine auxotroph phenotype, resistance to L-lysine analogue, and resistance to α-aminobutyric acid, and L-threonine producibility.

The *fhuC, fhuD,* and *fhuB* genes to be deleted were deleted from *E. coli* KCCM10812P and *E. coli* KCCM10541P in the same manner as in Example 1, respectively. As a result, an L-threonine producing strain, KCCM10541_AfhuCDB and an L-tryptophan producing strain, KCCM10812P_ΔfhuCDB were prepared.

### Example 6: Measurement of ATP Levels in L-Threonine Producing Strain and L-Tryptophan Producing Strain Having Inactivation of Proteins Encoded by fhuC, fhuD, and fhub Genes

In this Example, the intracellular ATP levels in the strains prepared in Example 5 were practically measured.

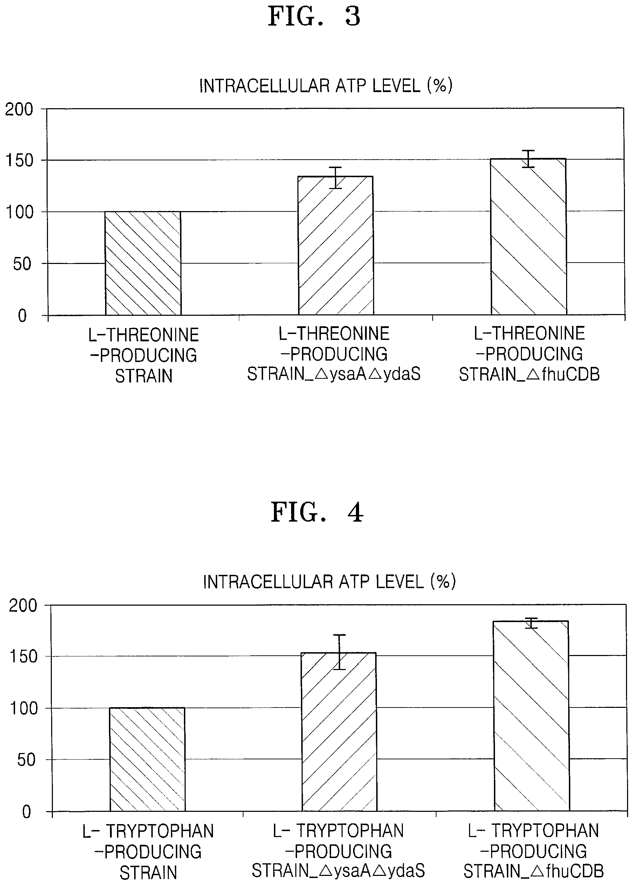

The intracellular ATP levels were measured in the same manner as in Example 2. The results are given in FIGS. 3 and 4. The results of FIGS. 3 and 4 were recorded as the average of three repeated experiments. As control groups, used were *ysa* and *ydaS-deleted,* L-threonine producing strain (*E. coli* KCCM10541P*_*Δ*ysa*Δ*ydaS*) and L-tryptophan producing strain (*E. coli* KCCM10812P-Δ*ysa*Δ*ydaS*) which are known to have higher intracellular ATP levels than the unmodified strains, *E. coli* KCCM10812P and *E. coli* KCCM10541P used in Example 3 (Korean Patent No. 1327093).

As shown in FIGS. 3 and 4, *fhuC, fhuD,* and *fhuB*-deleted strains prepared from the L-threonine producing strain and the L-tryptophan producing strain in Example 3 showed increased intracellular ATP levels, compared to the unmodified strains and control strains.

### Example 7: Examination of Titer of L-Threonine-Producing Strain Having the Inactivated Proteins Encoded by fhuC, fhuD, and fhub Genes

As described in Example 5, the strains with improved intracellular ATP levels, which were prepared by deletion of *fhuC, fhuD,* and *fhuB* genes in an L-threonine producing microorganism, *E. coli* KCCM10541P (Korean Patent No. 0576342), were subjected to titration using glucose as a carbon source. The *ysa* and *ydaS*-deleted L-threonine producing strain (E. coli KCCM10541P_Δ*ysa*Δ*ydaS*) was used as a control group to compare the titration results.

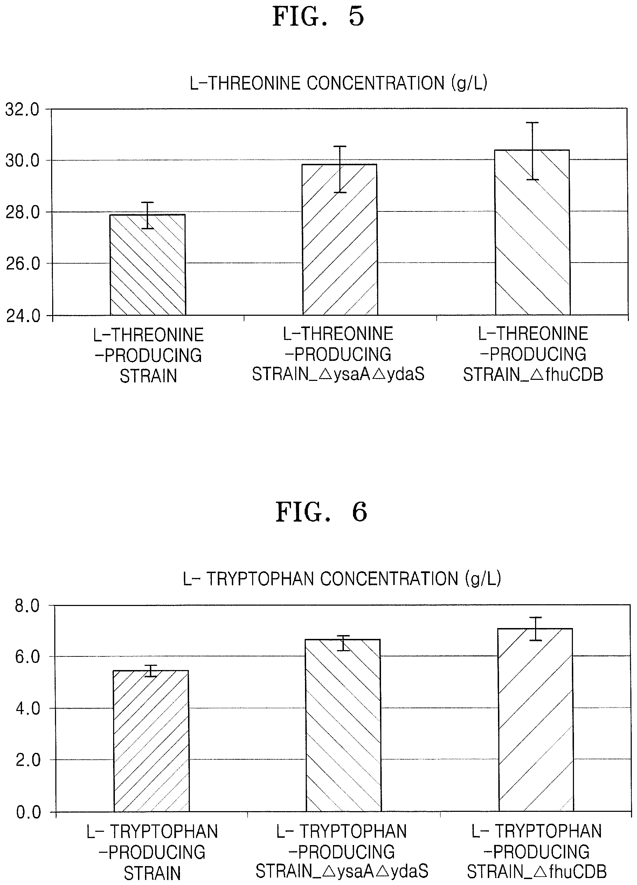

Each of the strains was cultured on an LB solid medium in an incubator at 33°C overnight, and then inoculated by a platinum loop into 25 mL of a glucose-containing titration medium containing the composition of Table 3. Then, the strains were cultured in an incubator at 33°C and at 200 rpm for 50 hours. The results are given in Table 4 and FIG. 5. All the results were recorded as the average of three repeated experiments.

**[Table 3]**

| Composition | Concentration (per liter) |
|---|---|
| Glucose | 70 g |
| KH₂PO₄ | 2 g |
| (NH₄)₂SO₄ | 25 g |
| MgSO₄·H₂O | 1 g |
| FeSO₄·H₂O | 5 mg |
| MnSO₄·H₂O | 5 mg |
| Yeast extract | 2 g |
| CaCO₃ | 30 g |

**[Table 4]**

| Strain | OD₅₆₂ | Glucose consumption (g/L)* | Production amount of L-threonine (g/L)** |
|---|---|---|---|
| KCCM10541P | 22.8 | 41.0 | 28.0 ± 0.5 |
| KCCM10541P_ΔvsaAΔydaS | 23.9 | 42.1 | 29.8 ± 0.9 |
| KCCM10541P_ΔfhuCDB | 23.1 | 41.8 | 30.5 ± 1 |

| | | | |
|---|---|---|---|
| * measured at 30 hours ** measured at 50 hours | | | |

As shown in Table 4, it was demonstrated that the recombinant L-threonine producing *E. coli* strain prepared according to the present application showed a physiological activity similar to that of the unmodified strain, and increased L-threonine production up to about 9%, compared to the unmodified strain. In view of the intracellular ATP levels confirmed in FIG. 3, these results indicate that L-threonine producibilities of the strains were increased by the increased intracellular ATP levels.

### Example 8: Examination of Titer of L-Tryptophan-Producinh Strain Having Inactivation of Proteins Encoded by fhuC, fhuD, and fhuB Genes

As described in Example 5, the strains with improved intracellular ATP levels, which were prepared by deletion of *fhuC, fhuD,* and *fhuB* genes in an L-tryptophan producing microorganism, KCCM10812P (Korean Patent No. 0792095), were subjected to titration using glucose as a carbon source. The *ysa* and *ydaS*-deleted L-tryptophan producing strain (E. coli KCCM10812P_Δ*ysa*Δ*ydaS*) was used as a control group to evaluate the titer in the same manner as in Example 4.

The titration results are given in Table 5 and FIG. 6. All the results were recorded as the average of three repeated experiments.

**[Table 5]**

| Strain | OD₆₀₀ | Glucose consumption (g/L)* | Production amount of L-tryptophan (g/L)** |
|---|---|---|---|
| KCCM10812P | 18.2 | 45.7 | 5.5 ± 0.2 |
| KCCM10812P_ΔysaAΔydaS | 18.3 | 46.3 | 6.7 ± 0.1 |
| KCCM10812P_ΔfhuCDB | 17.9 | 47.4 | 7.1 ± 0.5 |

| | | | |
|---|---|---|---|
| * measured at 33 hours ** measured at 48 hours | | | |

As shown in Table 5, it was demonstrated that the recombinant L-tryptophan producing *E. coli* strain prepared according to the present application showed a physiological activity similar to that of the unmodified strain, and increased L-tryptophan production up to about 30%, compared to the unmodified strain. In view of the intracellular ATP levels confirmed in FIG. 4, these results indicate that L-tryptophan producibilities of the strains were increased by the increased intracellular ATP levels.

The recombinant strain of the present application, CA04-2801(KCCM10812P_ΔfhuCDB) was deposited at the Korean Culture Center of Microorganisms, an international depository authority, on Nov. 15, 2013 under Accession NO. KCCM11474P.
## Claims

1. A method for producing L-amino acids, the method comprising:
culturing a microorganism of the genus *Escherichia* in a media, and
recovering L-amino acids from the culture media or the microorganism;
wherein the microorganism has an increased intracellular ATP level, compared to an unmodified strain, wherein one or more proteins selected from an amino acid sequence of SEQ ID NO: 5, an amino acid sequence of SEQ ID NO: 6, and an amino acid sequence of SEQ ID NO: 7, which constitute an iron uptake system, are inactivated in the microorganism;
wherein the microorganism of the genus *Escherichia* has an improved ability to produce L-amino acid, compared to an unmodified strain; and
wherein the L-amino acid is L-threonine or L-tryptophan.

2. The method according to claim 1, wherein all of the proteins having amino acid sequences of SEQ ID NOS: 5, 6, and 7 are inactivated.

3. The method according to claim 1, wherein the microorganism is *E. coli.*

4. The method according to claim 1, wherein the one or more proteins are inactivated by mutation due to deletion, substitution, or insertion of part or all of the gene encoding the corresponding protein, by modification of an expression regulatory sequence to reduce the expression of the gene, by modification of the chromosomal gene sequence to weaken or eliminate the activity of the protein, or by combinations thereof.

## Patentansprüche

1. Verfahren zum Herstellen von L-Aminosäuren, wobei das Verfahren Folgendes umfasst:
Kultivieren eines Mikroorganismus der Gattung *Escherichia* in einem Medien und Gewinnen von L-Aminosäuren aus den Kulturmedien oder dem Mikroorganismus;
wobei der Mikroorganismus einen erhöhten intrazellulären ATP-Spiegel im Vergleich zu einem unmodifizierten Stamm aufweist, wobei ein oder mehrere Proteine, die aus einer Aminosäuresequenz der SEQ ID NO: 5, einer Aminosäuresequenz der SEQ ID NO: 6 und einer Aminosäuresequenz der SEQ ID NO: 7 ausgewählt sind, die ein Eisenaufnahmesystem darstellen, in dem Mikroorganismus inaktiviert sind;
wobei der Mikroorganismus der Gattung *Escherichia* im Vergleich zu einem unmodifizierten Stamm eine verbesserte Fähigkeit zum Herstellen von L-Aminosäure aufweist; und
wobei die L-Aminosäure L-Threonin oder L-Tryptophan ist.

2. Verfahren nach Anspruch 1, wobei alle Proteine, die Aminosäuresequenzen der SEQ ID NO: 5, 6 und 7 aufweisen, inaktiviert werden.

3. Verfahren nach Anspruch 1, wobei der Mikroorganismus *E*. *coli* ist.

4. Verfahren nach Anspruch 1, wobei das eine oder die mehreren Proteine durch Mutation aufgrund von Deletion, Substitution oder Einfügung eines Teils oder des gesamten Gens, das für das entsprechende Protein kodiert, durch Modifikation einer Expressionsregulationssequenz, um die Expression des Gens zu verringern, durch Modifikation der Chromosomengensequenz, um die Aktivität des Proteins zu schwächen oder zu beseitigen, oder durch Kombinationen davon inaktiviert werden.

## Revendications

1. Procédé de production d'acides L-aminés, le procédé consistant à :
cultiver un microorganisme du genre *Escherichia* dans un milieu et récupérer les acides aminés L du milieu de culture ou du microorganisme ;
dans lequel le microorganisme a un taux d'ATP intracellulaire accru par rapport à une souche non modifiée, dans lequel une ou plusieurs protéines sélectionnées parmi une séquence d'acides aminés de SEQ ID NO: 5, une séquence d'acides aminés de SEQ ID NO: 6 et une séquence d'acides aminés de SEQ ID NO: 7, qui constituent un système d'absorption de fer, sont inactivés dans le microorganisme ;
dans lequel le microorganisme du genre *Escherichia* a une capacité améliorée à produire un acide L-aminé, par rapport à une souche non modifiée ; et
dans lequel le L-aminoacide est la L-thréonine ou le L-tryptophane.

2. Procédé selon la revendication 1, dans lequel toutes les protéines ayant des séquences d'acides aminés de SEQ ID NOS: 5, 6 et 7 sont inactivées.

3. Procédé selon la revendication 1, dans lequel le microorganisme est l'*E*. *coli.*

4. Procédé selon la revendication 1, dans lequel la ou les protéines sont inactivées par mutation due à la délétion, substitution ou insertion de la totalité ou d'une partie du gène codant la protéine correspondante, par modification d'une séquence régulatrice de l'expression afin de réduire l'expression du gène, par modification de séquence du gène chromosomique pour affaiblir ou éliminer l'activité de la protéine, ou par des combinaisons de celles-ci.
